(19) Europäisches Patentamt
European Patent Office
Office européen des brevets

(11) Publication number : 0 401 517 B1

(12) # EUROPEAN PATENT SPECIFICATION

(45) Date of publication of patent specification : **03.11.93 Bulletin 93/44**

(51) Int. Cl.$^5$ : **C07C 39/18,** C07C 39/205, C07C 43/23, C07C 69/734, A61K 31/05

(21) Application number : **90108515.9**

(22) Date of filing : **07.05.90**

(54) **Substituted 2-alkynylphenols with anti-inflammatory action, a process for their preparation and pharmaceutical compositions thereof.**

(30) Priority : **05.06.89 IT 2078489**

(43) Date of publication of application : **12.12.90 Bulletin 90/50**

(45) Publication of the grant of the patent : **03.11.93 Bulletin 93/44**

(84) Designated Contracting States : **AT BE CH DE DK ES FR GB GR IT LI LU NL SE**

(56) References cited :
**EP-A- 0 202 529**
**GB-A- 1 598 418**
**PATENT ABSTRACTS OF JAPAN, unexamined applications, C section, vol. 2, no. 131, October 31, 1978 THE PATENT OFFICE JAPANESE GOVERNMENT page 2822 C 78**

(73) Proprietor : **CAMILLO CORVI S.p.A.**
**via S. Marco 18**
**Milan (IT)**

(72) Inventor : **Pellegata, Renato**
**Via Cenisio, 18**
**Milan (IT)**
Inventor : **Luzzani, Franco**
**Via Foppa 25**
**Milan (IT)**
Inventor : **Zuccari, Giuseppe**
**Vis A. Bassi 9, S. Angelo Lodigiano**
**Milan (IT)**

(74) Representative : **Klausner, Erich et al**
**c/o Ufficio Internazionale Brevetti Ing. C. Gregorj S.p.A. Via Dogana 1**
**I-20123 Milano (IT)**

EP 0 401 517 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted 2-alkinylphenols that are useful as anti-inflammatory agents.

These new products are active in vitro as inhibitors of cyclo-oxygenase (malondialdehyde production test in guinea pig lung homogenates) and/or of the 5-lipo-oxygenase (A23187 stimulated rabbit leucocytes test).

It is now generally recognized that both the cyclo-oxygenase metabolites (e.g. prostaglandins) and those of lipo-oxygenase (e.g. the monohydroxyeicosatetetraenoic acids, 5-HETE, and the dihydroxyeicosatetetraenoic acids, 5,12-HETE) play an important role in the inflammatory processes. Therefore it is presumable that drugs inhibiting both metabolic routes of arachidonic acid exhibit a more selective type of anti-inflammatory action. In fact, dual inhibitors, such as BW 775c (Higgs, G.A., et al. - Biochem. Pharmacol. 28; 1959-1961, 1979, timegadine (Bramm, E., et al. - Agents and Actions 11; 402-409, 1981), KME-4 (Hidaka, T., et al. - Japan J. Pharmacol. 36; 77-85, 1984) have shown an unusual profile of anti-inflammatory effects.

Moreover, since the leukotrienes $C_4$, $_{D4}$, $_{E4}$, which are also 5-lipo-oxygenase derivatives, have been recognized to be important mediators in bronchial asthma and in the anaphylactic phenomena, selective inhibitors of the 5-lipo-oxygenase might be beneficial in the treatment of allergic asthma.

It is a further object of the present invention to provide appropriate processes for the preparation of these compounds and pharmaceutically acceptable compositions containing a therapeutically effective amount of the active compound for the treatment of various inflammatory conditions.

More specifically, the compounds of the present invention correspond to the following formula:

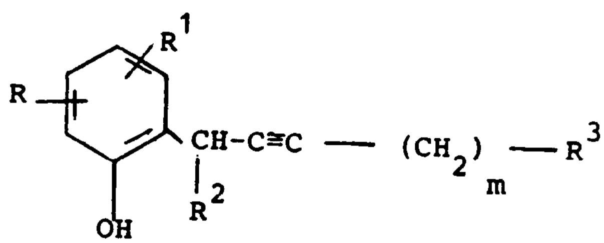

(I)

wherein

R and $R^1$ are selected independently from: hydrogen, hydroxyl, halogen, $(C_1 - C_6)$ alkyl, $(C_1 - C_4)$ alkoxy, benzyloxy, $-COOR^4$;

$R^2$ is : hydrogen, hydroxyl, $(C_1 - C_6)$alkyl, $R-C_6H_4-$;

$R^3$ is a methyl, R-aryl, heteroaryl, especially furyl and thienyl, $-COOR^4$ group;

$R^4$ is: hydrogen, $(C_1 - C_6)$ alkyl, $R-C_6H_4-$;

m is an integer of from 0 to 5.

The term "aryl" includes e.g. a phenyl or naphthyl group.

The term $(C_1 - C_6)$ alkyl includes a methyl, ethyl, n-propyl or isopropyl group, or a straight or branched chain butyl, pentyl or hexyl group.

The term "$(C_1 - C_4)$ alkoxy" includes a methoxy, ethoxy, n-propoxy or isopropoxy group or a straight or branched chain butoxyl group.

In a preferred embodiment of the present invention, the substituents in formula (I) have the following meanings:

R and $R^1$: hydrogen, $(C_1 - C_4)$ alkoxy, benzyloxy;

$R^2$ hydrogen, methyl;

$R^3$ : methyl, phenyl;

m is an integer of from 0 to 5; with the proviso that when $R^3$ is phenyl, then m is 0.

The term "$(C_1 - C_4)$ alkoxy" has the above mentioned meaning.

The compounds of formula (I) can be prepared by means of the reactions and processes described hereinafter. The reactions are usually carried out in a solvent appropriate for the reagents and materials employed, suitable for the conversions to be carried out. Unless otherwise stated, the reactions are carried out at the temperature of from -78°C and the boiling point of the solvents employed, so as to provide a reasonable reaction rate and an equally satisfactory stability of the reagents, solvents and materials involved. Sometimes the preparation of certain compounds of formula (I) by the method described requires that the functional groups in the starting materials be protected by blocking groups known per se from the chemical literature. These cases will be readily recognized by the skilled in the art. Illustrative examples of suitable blocking groups include benzyl ethers, methyl ethers and methoxymethyl ethers, for the protection of the hydroxyl groups, and trialkyl orthoesters for the protection of the ester group. The choice of the blocking groups is affected by the type of reaction

that is to be carried out on the material to be protected, as well as by the nature of the functional moieties present in other sites of the molecule that has to be stable under the conditions used for the removal of the blocking group after it has served its purpose. Where a blocking group is required, this group must often be removed in a separate step, after having performed those process steps with which the non-protected functional moiety is not compatible.

A reaction scheme according to the present invention is shown herein below:

Step A

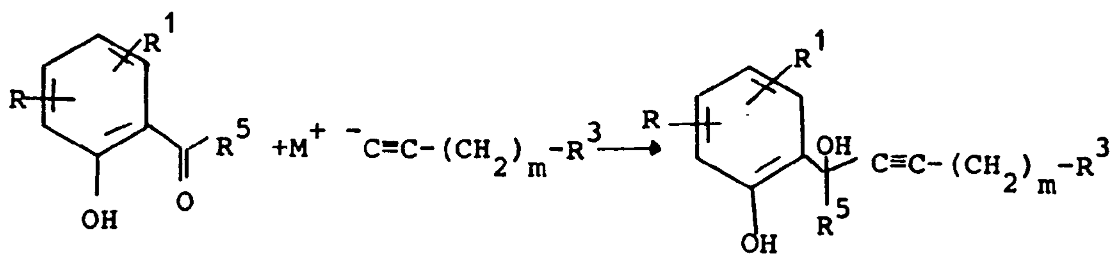

(II) (III) (IV)

wherein

R, $R^1$, $R^3$ and m have the above-mentioned meaning,

$R^5$ is hydrogen, $(C_1 - C_6)$ alkyl, $R\text{-}C_6H_4\text{-}$,

M is an alkali or earth-alkali metal such as sodium, potassium or lithium.

This reaction is carried out in the presence of a suitable organic solvent (e.g. tetrahydrofuran), by first reacting - preferably at low temperature (e.g. -78°C, -75°C) - the corresponding alkyne with a reagent that is able to convert it into the corresponding acetylide (III) e.g. butyllithium or sodium hydride), subsequently adding a suitable carbonyl compound (II) and then allowing the the temperature of the mixture to reach room temperature for the purpose of favouring the completion of the reaction.

Step B

$$\text{(IV)} \xrightarrow{Et_3SiH/BF_3\ .\ Et_2O} \text{(I)}$$

The reaction is carried out in the presence of a suitable organic solvent (e.g. methylenechloride) first at low temperature (e.g. -20°C) and then at room temperature for the purpose of completing the reaction.

The starting materials for the above described process steps are either commercially available or described in the chemical literature, or can be prepared from known compounds by routes described in the chemical literature. In particular, substituted terminal arylacetylenes can be prepared with the method described by E.I. Corey and P.L. Fuchs, TETRAHEDRON LETTERS, 3769, 1972, according to which the corresponding aldehyde is treated with the $CBr_4\text{-}PPh_3$ reagent and then with a strong base, such as n-butyl-lithium, to provide the desired intermediate product: lithium acetylide. Alternatively, the above referred starting materials can be prepared by reacting an aromatic halide with ethinyltrimethylsilane in the presence of palladium (O) (generated in situ), followed by the removal of the blocking group, as described by W.B. Austin, JOURNAL OF ORGANIC CHEMISTRY, 46, 2280, 1981.

For the treatment of inflammatory pathologies, rheumatoid arthritis, allergic conditions, psoriasis, asthma and other prostaglandin and/or leukotriene mediated disorders, a compound of formula (I) can be administered systemically (orally or parenterally) or topically, in a therapeutically effective amount; preferably, it will be administered in unit dose formulations containing conventional non toxic pharmaceutically acceptable carriers, adjuvants or vehicles. The term "parenteral" as used herein includes subcutaneous, intravenous, intramuscular injections or infusions.

The pharmaceutical compositions containing the active ingredient of the present invention can be in the form of tablets, capsules, aqueous or oily solutions, suspensions, dispersible powders or granules, tinctures, aerosol emulsions, creams, ointments, gels, suppositories or the like. These compositions can be prepared according to any method known to the pharmaceutical industry; moreover they can contain, besides a therapeutically effective amount of the compound of formula (I), also other known therapeutically active compounds. When the compound of the invention is administered systemically, it is preferably done so orally: through this route, the drug can be administered in the usual pharmaceutical formulations, such as tablets, capsules, solutions, suspensions or powders in a contemporaneous delivery or sustained release form. Any number of the usual excipients or tableting aids can likewise be included.

For topical administration, the compound of the invention or a pharmaceutically acceptable salt thereof is formulated as a topical preparation such as solutions, suspensions, powders, tinctures, aerosol emulsions, creams, ointments, sprays, gels and suppositories.

Doses of from 0.2 mg to 100 mg per kg of body weight per day can be used for the treatment of the abovementioned pathological conditions (i.e. usually of from 10mg to 5g per patient per day).

The specific dosage level for any particular patient depends however on a variety of factors including the intrinsic activity of the administered compound, the age, body weight, state of health, sex, diet, period of administration, route of administration, rate of excretion, concurrent administration of other drugs and severity of the illness.

BIOCHEMICAL DATA CONCERNING THE THERAPEUTICAL ACTIVITY OF THE COMPOUNDS OF FORMULA (I) IN VITRO ACTIVITY ON CYCLO-OXYGENASE AND 5-LIPOOXYGENASE

The cyclooxygenase activity is determined according to the method of Luzzani et al. (Luzzani, F., et al. INT. J. TISS. REAC. X(2); 79-83, 1988), by monitoring the production of malondialdehyde in a guinea pig lung homogenate incubated in the presence of arachidonic acid and cofactors.

The formation of leukotriene $B_4$ from [$^{14}C$] arachidonic acid in a preparation of calcium ionophore A23187 stimulated rabbit leukocites is measured by means of a slightly modified radiometric T.L.C.assay based on published procedures (Beetens, J.R. et al. BIOCHEM. PHARMACOL 35 (6); 883, 1986).

In Table I below the results of the comparisons of 8 inventive compounds with 3 prior art compounds are shown. In column "CO" the data concerning cyclo-oxygenase, and in column "5-LO" those concerning 5-lipooxygenase are shown.

TABLE I

| COMPOUND | CO | 5-LO |
|---|---|---|
| | (% of inhibition at 10 μM) | |
| CO/1827 | 71 | 15 |
| CO/1872 | 72 | 48 |
| CO/1826 | 66 | 52 |
| CO/1828 | 50 | 57 |
| CO/1863 | 23 | 46 |
| CO/1837 | 52 | 92 |
| CO:1841 | 26 | 90 |
| CO /1864 | 0 | 68 |
| INDOMETHACIN | 98 | 0 |
| BW 755 c | 30 | 25 |
| NORDIHYDROGUAIARETIC ACID | 5 | 92 |

The following examples serve to better illustrate the present invention.

EXAMPLE 1

Stage A:2-(3-Phenyl-1-hydroxy-2-propynyl)-4-methoxyphenol

A solution of phenylacetylene (1.1 ml = 10 mM) in anhydrous tetrahydrofuran (20 ml) is stirred at -78°C under nitrogen and treated with n-butyllithium (1.6 M in hexane: 6.25 ml = 10 mM). The solution is stirred for 30 minutes, then it is treated with a solution of 2-hydroxy-5-methoxybenzaldehyde (0.62 ml = 5 mM) in tetrahydro-furan (15 ml). The reaction mixture is brought to room temperature and stirred for 2 hours followed by slow addition of 50 ml 0.5 N hydrochloric acid, and the aqueous mixture is extracted with ethyl ether (3 x 50 ml). The combined organic extracts are washed with a saturated ammonium sulfate solution, anhydrated with $Na_2SO_4$ and concentrated to afford a product that is purified by flash-chromatography (3:1, n-hexane: ethyl acetate as the eluent). The residue is triturated with n-hexane to provide 1.22 g (96%) of a white solid, m.p. 64-66°C.

N.M.R. ($CDCl_3$): δ 7.55 - 6.90 (6H; aromatic; 6.90 - 6.55 (2H; aromatic); 5.78 (s; 1H; CH-C≡C); 3.65 (s; 3H; $CH_3$).

Stage B: 2-(3-Phenyl-2-propynyl)-4-metoxyphenol (CO/1828)

To a 48% solution of boron trifluoride etherate (8ml = 30 mM) in methylene chloride (100 ml) at -20°C, triethylsilane (10 ml = 63 mM) is added, to which is then added dropwise a solution of the compund described in stage A (2.5 g = 10 mM) in dichloromethane (20 ml).

The mixture is stirred for 30 minutes, then it is poured into a saturated sodium bicarbonate aqueous solution. The crude product is isolated by extraction with ethyl ether and purified by flash chromatography (6:1,

n-hexane: ethyl acetate as the eluent). The solid obtained is recrystallized from n-hexane to afford 1.26 g (53%) of the title compound as a white solid, m.p. 62-64°C.

N.M.R. ($CDCl_3$): δ 7.55 - 7.10 (5H; aromatic; 7.10 - 6.95 (1H; aromatic); 6.85 - 6.55 (2H; aromatic); 3.72 (s; 2H; $CH_2$-C≡C); 3.70 (s; 3H; $CH_3$).

EXAMPLE 2

Stage A: 2-(4-Phenyl-2-hydroxy-3-butyne-2-yl)-4-methoxyphenol

By a method similar to that described in Example 1, stage A, but using phenyl acetylene and 2-hydroxy-5-methoxyacetophenone as the starting compounds, the title compound is prepared with 94% yield as colourless oil .

Stage B: 2-(4-Phenyl-3-butyne-2-yl)-4-methoxyphenol CO/1863)

By using the procedure of Example 1, stage B, the compound of stage A is converted into the title compound with a 58% yield: a white solid, m.p. 46-49°C.

N.M.R. ($CDCl_3$): δ 7.50 - 7.15 (5H; aromatic); 6.95 (d(2Hz); 1H; aromatic); 6.80 - 6.50 (2H; aromatic); 4.13 (q; 1H; CH - C ≡C); 3.72 (s; 3H; $OCH_3$); 1.38 (d; 3H; $CH_3$ - C).

EXAMPLE 3

Stage A: 2-(1-Hydroxy-2-nonynyl)-4-methoxyphenol.

By a method similar to that described in Example 1, stage A, but using 1-octyne and 2-hydroxy-5-methoxybenzaldehyde as the starting compounds, the title compound is prepared with a 70% yield as a colourless oil.

Stage B: 4-Methoxy-2-(2-nonynyl)phenol CO/1837)

By using the procedure of Example 1, stage B, the compound of stage A is transformed into the title compound with a 64% yield; a colourless oil.

N.M.R. ($CDCl_3$): δ 6.85 (d(2,5 Hz); 1H; aromatic); 6.75 - 6.45 (2H; aromatic); 3.70 (s; 3H; $OCH_3$); 3.47 (t(2.5 Hz); 2H; Ph$\underline{CH_2}$-C≡C); 2.30 - 2.00 (m; 2H; C≡C-$\underline{CH_2}$-$CH_2$); 1.70 - 0.70 (11H; $(CH_2)_4$-$CH_3$).

EXAMPLE 4

Stage A: 2-(1-Hydroxy-2-hexynyl)-4-methoxyphenol

By a method like that described in Example 1 stage A, but using 1-pentyne and 2-hydroxy-5-methoxybenzaldeyde as the starting compounds, the title compound is prepared with a 98% yield as a colourless oil.

Stage B: 2-(2-Hexynyl)-4-methoxyphenol CO/1872)

By using the procedure of Example 1, stage B, the compound of stage A is converted into the title compound with a 50% yield: a colourless oil.

N.M.R. ($CDCl_3$): δ 6.90 (d(2 Hz); 1H; aromatic); 6.70 - 6.45 (2H; aromatic); 3.70 (s; 3H; $OCH_3$); 3.47 (t(2.5 Hz); 2H; Ph-$\underline{CH_2}$-C≡C); 2.20-2.00 (m; 2H; C≡C-$\underline{CH_2}$-$CH_2$); 1.75-1.25 (m; 2H; $\underline{CH_2}CH_3$); 0.97 (t; 3H; $\underline{CH_3}$-$CH_2$).

EXAMPLE 5

Stage A: 2-(4-Phenyl-2-hydroxy-3-butyne-2-yl)-5-methoxyphenol

By a method like that described in Example 1, stage A, but using phenylacetylene and 2-hydroxy-4-methoxyacetophenone as the starting compounds, the title compound is prepared with a 74% yield as a crude purple coloured oil.

Stage B: 2-(4-Phenyl-3-butyne-2-yl)-5-methoxyphenol CO/1842)

By using the procedure of Example 1, stage B, the compound of stage A is converted into the title compound with a 31% yield: a white solid, m.p. 32-34°C.

N.M.R. ($CDCl_3$) : δ 7.60 - 7.10 (6H; aromatic); 6.43 (dd(9 Hz and 2 Hz) 1H; aromatic); 4.05 (q; 1H; CH-C≡C); 3.70 (s; 3H; $OCH_3$); 1.53 (d; 3H; $\underline{CH_3}$ - CH)

EXAMPLE 6

Stage A: 2-(3-Phenyl-1-hydroxy-2-propynyl)-6-methoxyphenol

By a method similar to that described in Example 1, stage A, but using phenylacetylene and 2-hydroxy-3-methoxybenzaldehyde as the starting compounds, the title compound is prepared with a 83% yield as a white solid with m.p. 77-78°C.

Stage B: 2-(3-Phenyl-2-propynyl)-6-methoxyphenol CO/1826)

By using the procedure of Example 1, stage B, the crude compound of stage A is converted into the title compound with a 34% yield: a white solid, m.p. 50-52°C.

N.M.R. ($CDCl_3$) : δ 7.55 - 6.95 (6H; aromatic); 6.95 - 6.60 (2H; aromatic); 3.80 (2s; 5H; $CH_2$ and $CH_3$).

EXAMPLE 7

Stage A: 2-(1-Hydroxy-2-nonynyl)-6-methoxyphenol

By a method similar to that described in Example 1, stage A, but using 1-octyne and 2-hydroxy-3-methoxybenzaldehyde as the starting compounds, the title compound is prepared with a 78% yield as a white solid, m.p. 48-49°C.

N.M.R. ($CDCl_3$) : δ 7.07 (dd(6 Hz and 4 Hz); 1H, aromatic); 6.90 - 6.65 (2H; aromatic); 5.70 (m; 1H; CH-C≡C); 3.78 (s; 3H; $COH_3$); 2.30 - 2.10 (m; 2H; $CH_2$-C≡C); 1.70 - 0.75 (11H; $(CH_2)_4$-$CH_3$

Stage B: 6-Methoxy-2-(2-nonynyl)phenol CO/1827)

By using the procedure of Example 1, stage B, the compound of stage A is converted into the title compound with a 75% yield: a colourless oil.

N.M.R. ($CDCl_3$) : δ 7.07 (dd(8 Hz and 3 Hz); 1H; aromatic); 6.90 - 6.60 (2H; aromatic); 3.77 (s; 3H; $OCH_3$); 3.52 (t(2.52 Hz); 2H; Ph-$CH_2$-C≡C); 2.35 - 2.05 (m; 2H; C≡C-$\underline{CH_2}$-$CH_2$); 1.70 - 0.70 (11H; $(CH_2)_4$ - $CH_3$)

EXAMPLE 8

Stage A: 2,5-Dibenzyloxybenzaldehyde

A mixture of 2,5-dihydroxybenzaldehyde (5.52 g = 40 mM), benzyl bromide (9.5 ml = 80 mM) and anhydrous potassium carbonate (16.6 g = 120 mM) in anhydrous acetone (120 ml) is refluxed for 6 h and then concentrated under reduced pressure; the residue is then taken up with water and extracted with ethyl ether. The ether phases are washed with a saturated potassium carbonate and a saturated ammonium sulfate solution, anhydrated ($Na_2SO_4$) and concentrated to afford an oil. The trituration with n-hexane - diisopropylether provides 10.6 g (83%) crude 2,5-dibenzyloxybenzaldehyde as a white solid, m.p. 74-80°C.

Stage B: 5-Benzyloxy-2-hydroxybenzaldehyde

To a solution of the product of stage A (9.6 g = 30 mM) in anhydrous acetonitrile (100 ml), under stirring, aluminium chloride (8 g = 60 mM) is slowly added. After stirring at room temperature overnight, the reaction mixture is poured into cooled 6N HCl (120 ml) and extracted with ethyl ether. The organic phases are washed with water and with a saturated ammonium sulfate solution, anhydrated ($Na_2SO_4$) and concentrated to provide a crude oil that is purified by flash-chromatography (98:2, n-hexane: ethyl acetate, as the eluent) to give 2.9 g (42%) of a pale yellow solid, m.p. 84-86°C.

N.M.R. ($CDCl_3$) : δ 9.77 (s; 1H; CHO); 7.50 - 6.75 (8H; aromatic); 5.00 (s; 2H. $OCH_2$).

Stage C: 4-Benzyloxy-2-(3-phenyl-1-hydroxy-2-propynyl)phenol

With the same procedure as described in Example 1, stage A, but using phenyl acetylene and the compound of stage B, the title compound is obtained with an 80% yield as a white solid, m.p. 105-108°C.
N.M.R. ($CDCl_3$) : δ 7.55 - 7.10 (11H; aromatic); 6.90 - 6.60 (2H; aromatic); 5.83 (s; 1H; CHC≡C); 4.92 (s; 2H. $OCH_2$)

Stage D: 4-Benzyloxy-2-(3-phenyl-2-propynyl)phenol (CO/1874)

Using the procedure of Example 1, stage B, the compound of stage C is converted, with a 40% yield, into the title compound: a white solid, m.p. 64-66°C.
N.M.R. ($CDCl_3$) : 7.50 - 6.50 (13H; aromatic); 4.94 (s; 2H; $OCH_2$); 3.70 (s; 2H; $CH_2$-C≡C)

EXAMPLE 9

Stage A: 5-Benzyloxy-2-hydroxyacetophenone

A mixture of 2,5-dihydroxyacetophenone (6.10 g = 40 mM), benzyl bromide (5 ml = 42 mM) and anhydrous potassium carbonate (5,6 g = 40 mM in anhydrous acetone (120 ml)) is heated under reflux for 5 hours. Then, the mixture is concentrated under reduced pressure, the residue is taken up with 1N HCl (120 ml) and extracted with ethyl ether. The combined extracts are washed with water and with a saturated ammonium sulfate solution, anhydrated ($Na_2SO_4$) and concentrated to give a crude solid compound that is then purified by flash-chromatography (4:1, n-hexane: ethyl acetate as the eluent) to give 8 g (82%) of a yellow solid, m.p. 63-65°C.
N.M.R. ($CDCl_3$) : δ 7.50 - 6.75 (8H; aromatic); 4.96 (s; 2H; $OCH_2$); 2.49 (s; 3H; $CH_3$).

Stage B: 4-Benzyloxy-2-(4-phenyl-2-hydroxy-3-butyne-2-yl)phenol

With the same procedure as in Example 1, stage A, but using phenylacetylene and the compound of stage A, the title compound is obtained, with a 93% yield, as a colourless oil.

Stage C: 4-Benzyloxy-2-(4-phenyl-3-butyne-2-yl)phenol (CO/1864)

Using the procedure of Example 1, stage B, the compound of stage B is converted, with a 56% yield, into the title compound: a colourless oil.
N.M.R. ($CDCl_3$) : δ 7.55 - 6.95 (11H; aromatic); 4.95 (s; 2H; $OCH_2$); 4.10 (q; 1H; CH-C≡C); 1.53 (d; 3H; $CH_3$).

EXAMPLE 10

Stage A: 2-(3-Phenyl-1-hydroxy-2-propynyl-phenol

With a similar method as that described in Example 1, stage A, but using phenylacetylene and salicylaldehyde, the title compound is prepared, with a 93% yield, in the form of a white solid; m.p. 82-84°C.
N.M.R. ($CDCl_3$) : δ 7.55 - 7.00 (7H; aromatic); 7.00 - 6.70 (2H; aromatic); 5.82 (s; 1H; CH-C≡C).

Stage B: 2-(3-Phenyl-2-propynyl)phenol (CO/1836)

Using the procedure of Example 1, stage B, the compound of Stage A is converted, with a 36% yield, into the title compound: a colourless oil.
N.M.R. ($CDCl_3$) : δ 7.60 - 6.60 (9H; aromatic); 3.77 (s; 2H; $CH_2$).

EXAMPLE 11

Stage A: 2-(1-Hydroxy-2-nonynyl)phenol

With a similar method as that described in Example 1, stage A, but using 1-octyne and salicylaldehyde,

the title compound is obtained, with a 98% yield, as a white solid; m.p. 28-30°C.

N.M.R. ($CDCl_3$) : δ 7.45 - 6.65 (4H; aromatic); 5.58 (t(2 Hz); 1H; CH-C≡C); 2.40 - 2.00 (m; 2H; $CH_2$-C≡C); 1.75 - 0.70 (11H; $(CH_2)_4$-$CH_3$).

Stage B: 2-(2-Nonynyl)phenol (CO/1855)

Using the procedure of Example 1, stage B, the compound of stage A is converted, with a 43% yield, into the title compound: a colourless oil.

N.M.R. ($CDCl_3$) : δ 7.35 - 6.70 (4H; aromatic); 3.50 (t(2.5 Hz); 2H; Ph-$\underline{CH_2}$-C≡C); 2.35 - 2.00 (m; 2H; C≡C-$\underline{CH_2}$ - $CH_2$); 1.8 - 0.75 (11H; $(CH_2)_4$-$CH_3$).

EXAMPLE 12

Stage A: 2-(4-Phenyl-2-hydroxy-3-butyne-2-yl)phenol

With a similar method as that described in Example 1, stage A, but using phenyl acetylene and 2-hydroxyacetophenone, the title compound is obtained, with a 92% yield, as a colourless oil.

Stage B: 2-(4-Phenyl-3-butyne-2-yl)phenol (CO/1835)

Using the procedure of Example 1, stage B, the compound of stage A is converted, with 52% yield, into the title compound: a colourless oil.

N.M.R. ($CDCl_3$) : δ 7.55 - 6.65 (9H; aromatic); 4.14 (q; 1H; CH-C≡C) 1.55 (d; 3H; $CH_3$).

EXAMPLE 13

Stage A: 3-(3-Phenyl-1-hydroxy-2-propynyl)pyrocatechol

With a similar method as that described in Example 1, stage A, but using phenylacetylene and 2,3-dihydroxy-benzaldehyde, the title compound is obtained, with a 59% yield, as a pale yellow solid, m.p. 80-82°C.

Stage B: 3-(3-Phenyl-2-propynyl)pyrocatechol (CO/1841)

Using the procedure of Example 1, stage B, the compound of stage A is converted, with a 32% yield, into the title compound: a white solid, m.p. 46-50°C.

N.M.R. ($CDCl_3$) δ 7.55 - 7.15 (5H; aromatic); 7.05 - 6.70 (3H; aromatic); 3.85 (s; 2H; $CH_2$).

EXAMPLE 14

Stage A: 7-Hydroxy-7-(2-hydroxy-5-methoxy)phenyl-5-methylheptynoate

With a similar method as that described in Example 1, stage A, but using 1,1,1-trimethoxy-5-hexyne (G. Just, C. Luthe, TETRAHEDRON LETTERS 23, 1331, 1982) and 2-hydroxy-5-methoxybenzaldehyde, the title compound is obtained, with a 60% yield, as a white powder, m.p. 72-73°C.

N.M.R. ($CDCl_3$) : δ 7.00 - 6.85 (m; 1H; aromatic); 6.85 - 6.57 (2H; aromatic); 5.70 - 5.50 (m; 1H; CH-C≡C); 3.70 (s; 3H; $OCH_3$); 3.62 (s; 3H; $OCH_3$); 2.53 - 2.17 (4H; C≡C-$CH_2$ + $CH_2COO$); 2.03 - 1.60 (m; ZH; $CH_2$-$\underline{CH_2}$-$CH_2$)

Stage B: 7-(2-Hydroxy-5-methoxy)phenyl-5-methylheptynoate (CO/1859)

Using the procedure of Example 1, stage B, the compound of stage A is converted, with a 35% yield, into the title compound: a colourless oil.

N.M.R. ($CDCl_3$) : δ 6.86 (d(2.5 Hz); 1H; aromatic); 6.80 - 6.50 (2H; aromatic); 3.73 (s; 3H, $OCH_3$); 3.65 (s; 3H; $OCH_3$); 3.48 (t(2.5 Hz); 2H; Ph-$\underline{CH_2}$ - C≡C); 2.43 (t; 2H; $CH_2COO$); 2.30 - 2.10 (m; 2H; C=C-$\underline{CH_2}$-$CH_2$); 2.00 - 1.65 (m; 2H; C≡C=$CH_2$-$\underline{CH_2}$)

EXAMPLE 15

Stage A: 4,6-Dichloro-2-(3-phenyl-1-hydroxy-2-propynyl)phenol

With a similar method as that described in Example 1, stage A, but using phenylacetylene and 3,5-dichlorosalicylaldehyde, the title compound is obtained, with a 66% yield, as a white solid, m.p. 90-92°C.

Stage B: 4,6-Dichloro-2-(3-phenyl-2-propynyl)phenol (CO/1899)

Using the procedure of Example 1, stage B, the compound of stage A is converted, with a 51% yield, into the title compound: a white solid, m.p. 40-42°C.
N.M.R. ($CDCl_3$) : δ 7.60 - 7.00 (7H; aromatic); 3.78 (s; 2H; $CH_2$-C≡C).

EXAMPLE 16

Stage A: 2-[1-Hydroxy-3-(3-methoxyphenyl)-2-propynyl]-4-methoxy-phenol

A solution of lithium 3-methoxyphenyl-acetylide in tetrahydrofuran (prepared according to Corey and Fuchs, starting from 3-methoxybenzaldehyde) is cooled to -78°C, then treated with a solution of 2-hydroxy-5-methoxybenzaldehyde in tetrahydrofuran with a similar method to that described in Example 1, stage A: the title compound is obtained, with a 32% yield, as a white solid, m.p. 92-94°C.

Stage B: 4-Methoxy-2-[3-(3-methoxyphenyl)-2-propynyl]phenol (CO/1910)

Using the procedure of Example 1, stage B, the compound of stage A is converted, with a 34% yield, into the title compound: a white solid, m.p. 51-53°C.
N.M.R. ($CDCl_3$) : δ 7.35 - 6.45 (7H; aromatic); 3.72 (br s; 8H; 2 $OCH_3$ and $CH_2$C≡C).

EXAMPLE 17

Stage A: 2-[1-Hydroxy-3-(2-naphthyl)-2-propynyl]-4-methoxyphenol

With a similar method as that described in Example 16, stage A, but starting from 2-naphthaldehyde, the title compound is obtained, with a 73% yield, as a solid, m.p. 112-115°C.

Stage B: 4-Methoxy-2-[3-(2-naphthyl)-2-propynyl]phenol (CO/1911)

Using the procedure of Example 1, stage B, the compound of stage A is converted, with a 52% yield, into the title compound: a pale brown solid, m.p. 67-70°C.
N.M.R. ($CDCl_3$) : δ 7.97 - 6.65 (10H, aromatic), 5.14 (s; 1H; OH); 3.79 (s; 2H; $CH_2$); 3.73 (s; 3H; $OCH_3$)

EXAMPLE 18

Stage A: 2-(1,3-Diphenyl-1-hydroxy-2-propynyl)phenol

With a similar method as that described in Example 1, stage A, but using phenylacetylene and 2-hydroxybenzophenone, the title compound is obtained, with a 92% yield, as a white solid, m.p. 114-116°C.

Stage B: 2-(1,3-Diphenyl-2-propynyl)phenol (CO/1912)

Using the procedure of Example 1, stage B, the compound of stage A is converted, with a 43% yield, into the title compound: a viscous yellow oil.
N.M.R. ($CDCl_3$) : δ 7.61 - 6.63 (14H; aromatic); 5.64 (s; 1H; CHC≡C); 5.46 (s; 1H; OH).

EXAMPLE 19

Stage A: 2-[3-(4-Chlorophenyl)-1-hydroxy-2-propynyl]-4-methoxyphenol

With a similar method as that described in Example 16, stage A, but starting from p-chlorobenzaldehyde, the title compound is obtained, with 65% yield, as a white solid, m.p. 103-105°C.

Stage B: 2-[3-(4-Chlorophenyl)-2-propynyl]-4-methoxyphenol (CO/1913)

Using the procedure of Example 1, stage B, the compound of stage A is converted, with 44% yield, into the title compound: a white solid, m.p. 110-115°C.

N.M.R. ($CDCl_3$ + DMSO) : δ 7.45-7.10 (4H; aromatic); 7.10 - 6.95 (d(2.5 Hz); 1H; aromatic); 6.85 - 6.45 (d and dd (2.5 and 8 Hz); 2H; aromatic; 3.75 (s; 2H; $CH_2$-C≡C); 3.72 (s; 3H; $OCH_3$).

EXAMPLE 20

A topical formulation, (a solution in distilled water) is prepared by dissolving a compound of formula (I) in a sufficient amount of water to achieve the desired concentration and by filtering the solution.

EXAMPLE 21

A topical formulation (tincture) is prepared by dissolving a compound of formula (I) in 50% U.S.P. alcohol, adding a sufficient amount of water to obtain the desired concentration and filtering.

EXAMPLE 22

A topical formulation (ointment) is prepared by heating U.S.P. Petrolatum to 60°C, adding a compound of formula (I), stirring until an thoroughly dispersed and then cooling to room temperature.

EXAMPLE 23

A topical formulation (aerosol) is prepared by dissolving a compound of formula (I) in U.S.P. alcohol and isopropylmyristate, adding conventional propellents until the desired concentration is obtained, and introducing into conventional aerosol containers.

**Claims**

**Claims for the following Contracting States : AT, BE, CH, DE, DK, FR, GB, IT, LI, LU, NL, SE**

1. A compound of formula

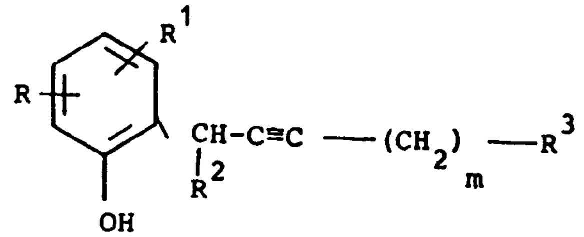

(I)

wherein

R and $R^1$ are chosen independently from: hydrogen, hydroxyl, halogen, ($C_1$ - $C_6$) alkyl, ($C_1$ - $C_4$) alkoxy, benzyloxy, -$COOR^4$;

$R^2$ is : hydrogen, hydroxyl, ($C_1$ - $C_6$) alkyl, R-$C_6$-$H_4$-;

$R^3$ is a methyl, R-aryl, heteroaryl, especially furyl and thienyl, -$COOR^4$ group;

$R^4$ is: hydrogen, ($C_1$ - $C_6$) alkyl, R-$C_6H_4$-;

m is an integer of from 0 to 5;
and a pharmaceutically acceptable salt thereof.

2. A compound of formula (I) according to claim 1 characterized in that the substituents have the following meanings:
R and $R^1$: hydrogen, $(C_1 - C_4)$ alkoxy, benzyloxy;
$R^2$: hydrogen, methyl;
$R^3$ : methyl, phenyl;
m is an integer of from 0 to 5; with the proviso that when $R^3$ is phenyl, then m is 0, and a pharmacologically acceptable salt thereof.

3. A process for the preparation of a compound of formula (I) according to claim 1 or 2, characterized in that a carbonyl compound of formula

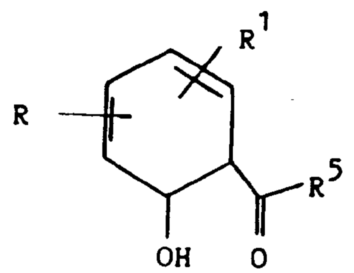

(II)

where R and $R^1$ are as defined in the preceding claims and $R^5$ is hydrogen, $(C_1 - C_6)$ alkyl, or R-$C_6H_4$-, is reacted at a temperature of from -78°C to the boiling temperature of the solvent(s) employed with an alkali metal salt of formula

$$M^+ \, ^-C \equiv C - (CH_2)_m - R^3 \qquad (III)$$

wherein $R^3$ is a methyl, R-aryl, heteroaryl, -$COOR^4$ group, $R^4$ being hydrogen, $(C_1 - C_6)$ alkyl, R-$C_6H_4$-; followed by reacting the compound of formula

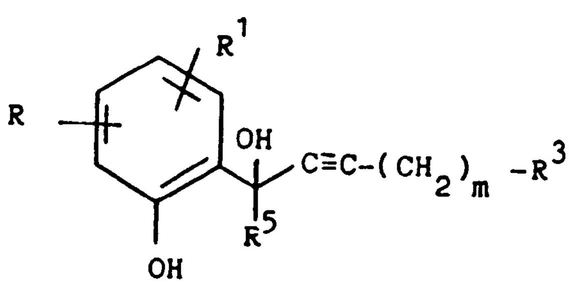

(IV)

thus obtained, wherein the substituents are as defined hereinabove, in the presence of an organic solvent, with a mixture of triethylsilane in ethered boron trifluoride, at a temperature from -25 to -15°C, then allowing the reaction mixture to reach the room temperature.

4. A process for the preparation of a compound of formula (I) according to claim 1 or 2, characterized in that the corresponding alkine is reacted in tetrahydrofuran, at a temperature of from -78°C to -75°C, with a reagent that can convert it into the acetylide

$$M^+ \, ^-C \equiv C - (CH_2)_m - R^3 \qquad (III)$$

wherein $R^3$ is a methyl, R-aryl, heteroaryl, -$COOR^4$ group, $R^4$ being hydrogen, $(C_1 - C_6)$ alkyl, R-$C_6H_4$-; whereupon a methoxyphenol of the formula

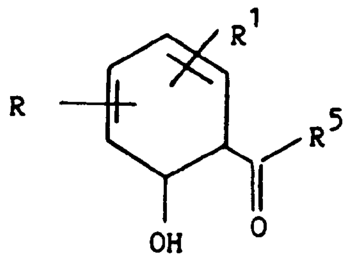

(II)

is added
wherein R and $R^1$ are as defined in the preceding claims and $R^5$ is hydrogen, ($C_1$ - $C_6$) alkyl, or R-$C_6H_4$-, then allowing the temperature of the reaction mixture to reach the room temperature, whereupon the compound

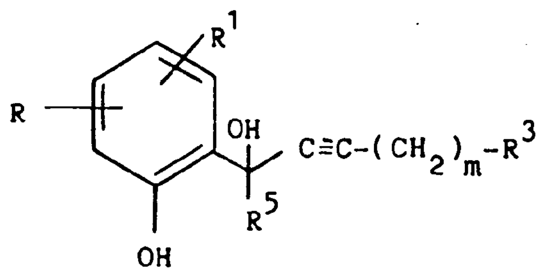

(IV)

thus obtained, in which the substituents are as defined above, is treated in the presence of methylene chloride, with a mixture of triethylsilane in ethered boron trifluoride, at a temperature from -25 to -15°C then allowing the reaction mixture to reach room temperature.

5. A pharmaceutical composition for the topical treatment of inflammatory conditions, characterized in that it contains, as the therapeutically active agent, a pharmacologically effective amount of at least one compound of formula (I) according to claim 1, or a pharmacologically acceptable salt thereof.

**Claims for the following Contracting State : ES**

1. A process for the preparation of a compound of formula

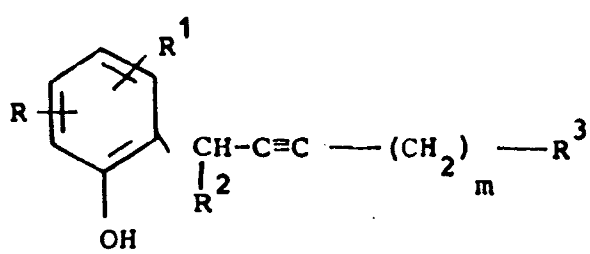

(I)

wherein
R and $R^1$ are chosen independently from : hydrogen, hydroxyl, halogen, ($C_1$ - $C_6$ ) alkyl, ($C_1$ - $C_4$) alkoxy, benzyloxy, -$COOR^4$ group ;
$R^2$ is : hydrogen, hydroxyl, ($C_1$ - $C_6$ ) alkyl, R-$C_6H_4$- ;
$R^3$is a methyl, R-aryl, heteroaryl, especially furyl and thienyl, -$COOR^4$group;
$R^4$ is hydrogen, ( $C_1$ - $C_6$) alkyl, R-$C_6H_4$-;
m is an integer of from 0 to 5 ;
or a pharmaceutically acceptable salt thereof, characterized in that a carbonyl compound of formula

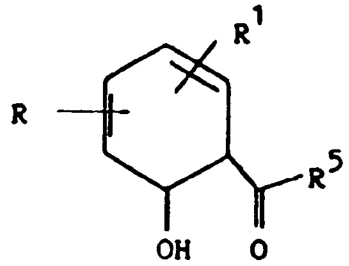

(II)

wherein R and $R^1$ are as defined hereinabove and $R^5$ is hydrogen, $(C_1 - C_6)$ alkyl, or $R\text{-}C_6H_4\text{-}$, is reacted at a temperature of from -78°C to the boiling temperature of the solvent(s) employed with an alkali metal salt of formula

$$M^+ {}^-C \equiv C - (CH_2)_m - R^3 \qquad (III)$$

wherein $R^3$ is a methyl, R-aryl, heteroaryl, $-COOR^4$ group, $R^4$ being hydrogen, $(C_1 - C_6)$ alkyl, $R\text{-}C_6H_4\text{-}$; followed by reacting the compound formula

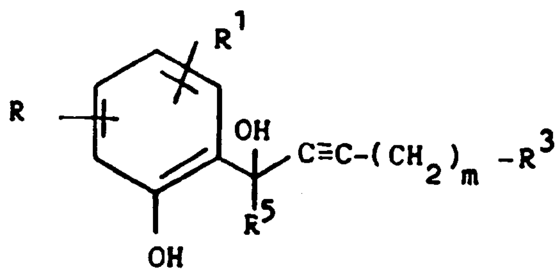

(IV)

thus obtained, wherein the substituents are as defined hereinabove, in the presence of an organic solvent, with a mixture of triethylsilane in ethered boron trifluoride, at a temperature of from -25 to -15°C, and then allowing the reaction mixture to reach room temperature.

2. A process for the preparation of a compound of formula I according to claim I
wherein
R and $R^1$ are chosen independently from : hydrogen, hydroxyl, halogen, $(C_1 - C_6)$ alkyl, $(C_1 - C_4)$ alkoxy, benzyloxy, $-COOR^4$;
$R^2$ is : hydrogen, hydroxyl, $(C_1 - C_6)$ alkyl, $R\text{-}C_6H_4\text{-}$;
$R^3$ is a methyl, R-aryl, heteroaryl, especially furyl and thienyl, $-COOR^4$ group;
$R^4$ is : hydrogen, $(C_1 - C_6)$ alkyl, $R\text{-}C_6H_4\text{-}$;
m is an integer of from 0 to 5;
and a pharmaceutically acceptable salt thereof, characterized in that the corresponding alkine is reacted in tetrahydrofuran, at a temperature of from 78°C to -75°C, with a reagent that can convert it into the acetylids

$$M^+ {}^-C \equiv C = (CH_2)_m - R^3 \qquad (III)$$

wherein $R^3$ is a methyl, R-aryl, heteroarlyl, $-COOR^4$ group, $R^4$ being hydrogen, $(C_1 - C_6)$ alkyl, $R\text{-}C_6H_4\text{-}$; whereupon a methoxyphenol of the formula

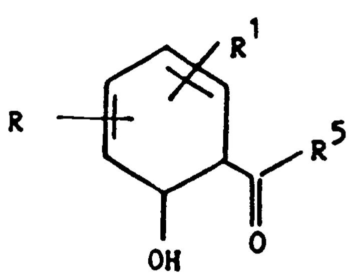

(II)

is added
wherein R and $R^1$ are as defined in claim 1, then allowing the temperature of the reaction mixture to reach

room temperature, whereupon the compound of formula

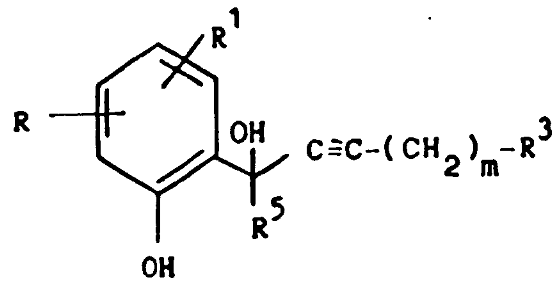

(IV)

thus obtained, in which the substituents are as defined above, is treated in the presence of methylene chloride, with a mixture of triethylsilane in ethered boron trifluoride, at a temperature of from -25 to -15°C, and then allowing the reaction mixture to reach room temperature.

3. A process for preparing a pharmaceutical composition for the topical treatment of inflammatory conditions, characterized in that a therapeutically active quantity of a compound according to claim 1 is admixed with a suitable pharmaceutically acceptable diluent, carrier and/or excipient.

**Claims for the following Contracting State : GR**

1. A compound of formula

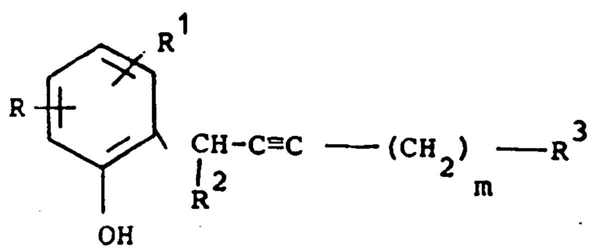

(I)

wherein
R and $R^1$ are chosen independently from: hydrogen, hydroxyl, halogen, ($C_1$ - $C_6$) alkyl, ($C_1$ - $C_4$) alkoxy, benzyloxy, -$COOR^4$;
$R^2$ is : hydrogen, hydroxyl, ($C_1$ - $C_6$) alkyl, R-$C_6H_4$-;
$R^3$ is a methyl, R-aryl, heteroaryl, especially furyl and thienyl, -$COOR^4$ group;
$R^4$ is: hydrogen, ($C_1$ - $C_6$) alkyl, R-$C_6H_4$-
m is an integer of from 0 to 5;
and a pharmaceutically acceptable salt thereof.

2. A compound of formula (I) according to claim 1 characterized in that the substituents have the following meanings:
R and $R^1$: hydrogen, ($C_1$ - $C_4$) alkoxy, benzyloxy;
$R^2$: hydrogen, methyl;
$R^3$ : methyl, phenyl;
m is an integer of from 0 to 5; with the proviso that when $R^3$ is phenyl, then m is 0, and a pharmacologically acceptable salt thereof.

3. A process for the preparation of a compound of formula (I) according to claim 1 or 2, characterized in that a carbonyl compound of formula

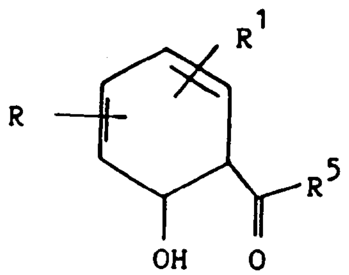

(II)

wherein R and $R^1$ are as defined in the preceding claims and $R^5$ is hydrogen, $(C_1 - C_6)$ alkyl, or $R\text{-}C_6H_4\text{-}$, is reacted at a temperature of from -78°C to the boiling temperature of the solvent(s) employed with an alkali metal salt of formula

$$M^+\,{}^-C \equiv C - (CH_2)_m - R^3 \qquad (III)$$

wherein $R^3$ is a methyl, R-aryl, heteroaryl, $-COOR^4$ group, $R^4$ being hydrogen, $(C_1 - C_6)$ alkyl, $R\text{-}C_6H_4\text{-}$; followed by reacting the compound of formula

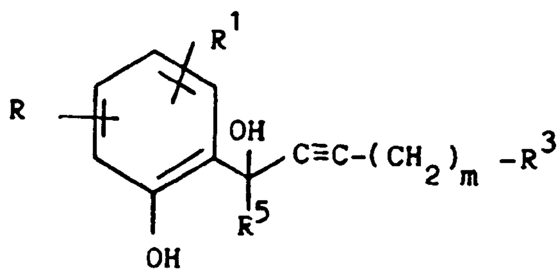

(IV)

thus obtained, wherein the substituents are as defined hereinabove, in the presence of an organic solvent, with a mixture of triethylsilane in ethered boron trifluoride, at a temperature from -25 to -15°C, then allowing the reaction mixture to reach room temperature.

4. A process for the preparation of a compound of formula (I) according to claim 1 or 2, characterized in that the corresponding alkine is reacted in tetrahydrofuran, at a temperature of from -78°C to -75°C, with a reagent that can convert it into the acetylide

$$M^+\,{}^-C \equiv C - (CH_2)_m - R^3 \qquad (III)$$

wherein $R^3$ is a methyl, R-aryl, heteroaryl, $-COOR^4$ group, $R^4$ being hydrogen, $(C_1 - C_6)$ alkyl, $R\text{-}C_6H_4\text{-}$; whereupon a methoxyphenol of the formula

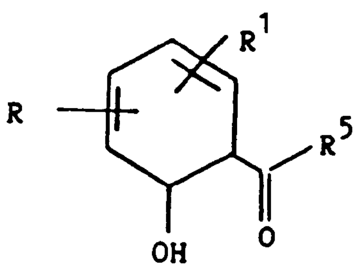

(II)

is added

wherein R and $R^1$ are as defined in the preceding claims and $R^5$ is hydrogen, $(C_1 - C_6)$ alkyl, or $R\text{-}C_6H_4\text{-}$, then allowing the temperature of the reaction mixture to reach the room temperature, whereupon the compound of formula

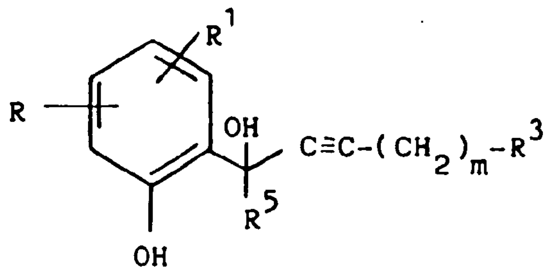

(IV)

thus obtained, in which the substituents are as defined above, is treated in the presence of methylene chloride, with a mixture of triethylsilane in ethered boron trifluoride, at a temperature from -25 to -15°C then allowing the reaction mixture to reach room temperature.

5. A process for preparing a pharmaceutical composition for the topical treatment of inflammatory conditions, characterized in that a therapeutically active quantity of a compound according to claim 1 is admixed with a suitable pharmaceutically acceptable diluent, carrier and/or excipient.

**Patentansprüche**

**Patentansprüche für folgende Vertragsstaaten : AT, BE, CH, DE, DK, FR, GB, IT, LI, LU, NL, SE**

1. Eine Verbindung der Formel

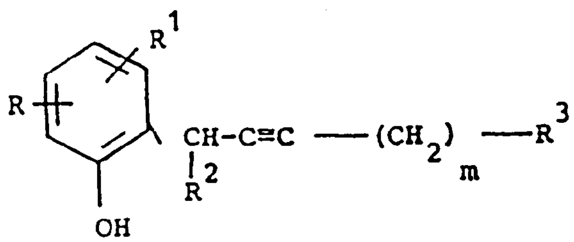

(I)

wobei
R und $R^1$ unabhängig voneinander ausgewählt werden aus Wasserstoff, Hydroxyl, Halogen, $(C_1 - C_6)$-Alkyl, $(C_1 - C_4)$-Alkoxy, Benzyloxy, -$COOR^4$;
$R^2$ ist : Wasserstoff, Hydroxyl, $(C_1 - C_6)$-Alkyl, R-$C_6H_4$-;
$R^3$ ist eine Methyl-, R-Aryl-, Heteroaryl-, besonders Furyl- und Thienyl-, -$COOR^4$-Gruppe;
$R^4$ ist: Wasserstoff, $(C_1 - C_6)$-Alkyl, R-$C_6H_4$-;
m ist eine ganze Zahl von 0 bis 5;
und ein pharmazeutisch annehmbares Salz davon.

2. Eine Verbindung der Formel (I) nach Anspruch 1, dadurch gekennzeichnet, daß die Substituenten die folgenden Bedeutungen haben:
R und $R^1$: Wasserstoff, $(C_1 - C_4)$-Alkoxy, Benzyloxy;
$R^2$: Wasserstoff, Methyl;
$R^3$: Methyl, Phenyl;
m ist eine ganze Zahl von 0 bis 5; mit der Maßgabe, daß, wenn $R^3$ Phenyl ist, m dann 0 ist;
und ein pharmakologisch annehmbares Salz davon.

3. Ein Verfahren zur Herstellung einer Verbindung der Formel (I) nach Anspruch 1 oder 2, dadurch gekennzeichnet, daß eine Carbonylverbindung der Formel

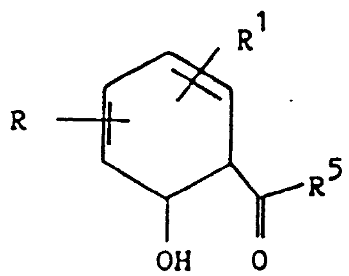

(II)

wobei R und $R^1$ wie in den vorhergehenden Ansprüchen definiert sind und $R^5$ Wasserstoff, ($C_1$ - $C_6$)-Alkyl oder R-$C_6H_4$- ist, bei einer Temperatur von -78°C bis zum Kochpunkt des verwendeten Lösungsmittels umgesetzt wird mit einem Alkalimetallsalz der Formel:

$$M^+ \, {}^-C \equiv C - (CH_2)_m - R^3 \qquad (III)$$

wobei $R^3$ eine Methyl-, R-Aryl-, Heteroaryl-, -$COOR^4$-Gruppe ist, wobei $R^4$ Wasserstoff, ($C_1$ - $C_6$)-Alkyl, R-$C_6H_4$- ist; gefolgt davon, daß man die Verbindung der Formel

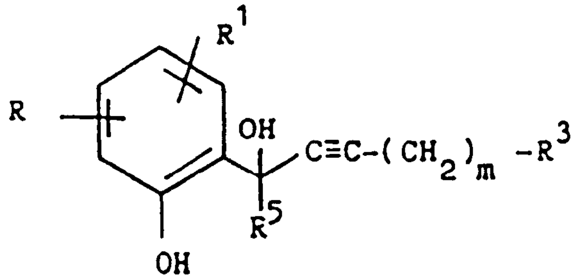

(IV)

die so erhalten wurde, wobei die Substituenten wie oben definiert sind, in Gegenwart eines organischen Lösungsmittels mit einer Mischung von Triethylsilan in etherischem Bortrifluorid bei einer Temperatur von -25° bis -15°C umsetzt, und man dann die Reaktionsmischung die Raumtemperatur erreichen läßt.

4. Ein Verfahren zur Herstellung einer Verbindung der Formel (I) nach Anspruch 1 oder 2, dadurch gekennzeichnet, daß das entsprechende Alkin in Tetrahydrofuran bei einer Temperatur von -78°C bis -75°C mit einem Reagenz umgesetzt wird, daß es umwandeln kann in das Acetylid

$$M^+ \, {}^-C \equiv C - (CH_2)_m - R^3 \qquad (III)$$

wobei $R^3$ eine Methyl-, R-Aryl-, Heteroaryl-, -$COOR^4$-Gruppe ist, wobei $R^4$ Wasserstoff, ($C_1$ - $C_6$)-Alkyl, R-$C_6H_4$- ist; wonach ein Methoxyphenol der Formel

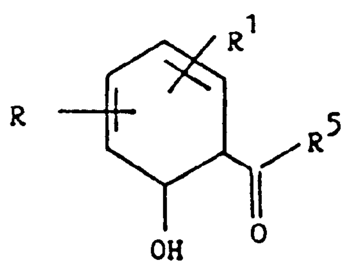

(II)

hinzugegeben wird,
wobei R und $R^1$ wie in den vorhergehenden Ansprüchen definiert sind und $R^5$ Wasserstoff, ($C_1$ - $C_6$)-Alkyl oder R-$C_6H_4$- ist, man dann die Temperatur der Reaktionsmischung auf Raumtemperatur ansteigen läßt, wonach man die Verbindung

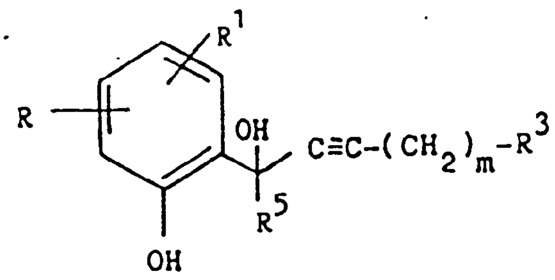

(IV)

die so erhalten wurde, bei der die Substituenten wie oben definiert sind, in Gegenwart von Methylenchlorid mit einer Mischung von Triethylsilan in etherischen Bortrifluorid bei einer Temperatur von -25° bis -15°C umsetzt, und man dann die Reaktionsmischung Raumtemperatur erreichen läßt.

5. Eine pharmazeutische Zusammensetzung zur topischen Behandlung von Entzündungszuständen, dadurch gekennzeichnet, daß sie als therapeutisch wirksames Mittel eine pharmakologisch wirksame Menge wenigstens einer Verbindung der Formel (I) nach Anspruch 1 oder ein pharmazeutisch annehmbares Salz davon enthält.

**Patentansprüche für folgenden Vertragsstaat : ES**

1. Ein Verfahren zur Herstellung einer Verbindung der Formel

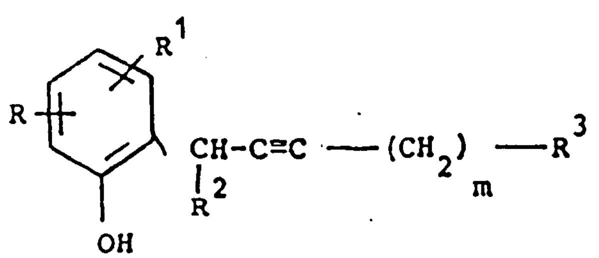

(I)

wobei
R und $R^1$ unabhängig voneinander ausgewählt werden aus Wasserstoff, Hydroxyl, Halogen, einer $(C_1 - C_6)$-Alkyl-, $(C_1 - C_4)$-Alkoxy-, Benzyloxy-, $-COOR^4$-Gruppe;
$R^2$ ist : Wasserstoff, Hydroxyl, $(C_1 - C_6)$-Alkyl, $R-C_6H_4-$;
$R^3$ ist eine Methyl-, R-Aryl-, Heteroaryl-, besonders Furyl- und Thienyl-, $-COOR^4$-Gruppe;
$R^4$ ist: Wasserstoff, $(C_1 - C_6)$-Alkyl, $R-C_6H_4-$;
m ist eine ganze Zahl von 0 bis 5;
oder eines pharmazeutisch annehmbaren Salzes davon, dadurch gekennzeichnet, daß eine Carbonylverbindung der Formel

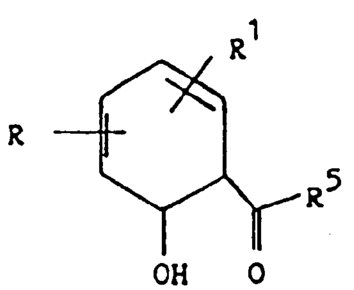

(II)

wobei R und $R^1$ wie oben definiert sind und $R^5$ Wasserstoff, $(C_1$-$C_6)$-Alkyl oder $R-C_6H_4-$ ist, bei einer Temperatur von -78°C bis zum Kochpunkt des verwendeten Lösungsmittels umgesetzt wird mit einem Alkalimetallsalz der Formel:

$$M^+ \, ^-C \equiv C - (CH_2)_m - R^3 \qquad (III)$$

wobei $R^3$ eine Methyl-, R-Aryl-, Heteroaryl-, $-COOR^4$-Gruppe ist, wobei $R^4$ Wasserstoff, $(C_1 - C_6)$-Alkyl,

$R-C_6H_4-$ ist; gefolgt davon, daß man die Verbindung der Formel

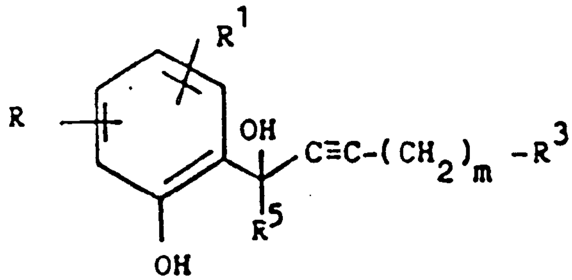

(IV)

die so erhalten wurde und wobei die Substituenten wie oben definiert sind, in Gegenwart eines organischen Lösungsmittels mit einer Mischung von Triethylsilan in etherischem Bortrifluorid bei einer Temperatur von -25° bis -15°C umsetzt, und man dann die Reaktionsmischung die Raumtemperatur erreichen läßt.

2. Ein Verfahren zur Herstellung einer Verbindung der Formel (I) nach Anspruch 1,
wobei
R und $R^1$ unabhängig voneinander ausgewählt werden aus Wasserstoff, Hydroxyl, Halogen, einer $(C_1 - C_6)$-Alkyl-, $(C_1 - C_4)$-Alkoxy-, Benzyloxy-, $-COOR^4$-Gruppe;
$R^2$ ist : Wasserstoff, Hydroxyl, $(C_1 - C_6)$-Alkyl, $R-C_6H_4-$;
$R^3$ ist eine Methyl-, R-Aryl-, Heteroaryl-, besonders Furyl- und Thienyl-, $-COOR^4$-Gruppe;
$R^4$ ist: Wasserstoff, $(C_1 - C_6)$-Alkyl, $R-C_6H_4-$;
m ist eine ganze Zahl von 0 bis 5;
und eines pharmazeutisch annehmbaren Salzes davon, dadurch gekennzeichnet, daß das entsprechende Alkin in Tetrahydrofuran bei einer Temperatur von -78°C bis -75°C mit einem Reagenz umgesetzt wird, daß es umwandeln kann in die Acetylid

$$M^+ \, ^-C \equiv C - (CH_2)_m - R^3 \qquad (III)$$

wobei $R^3$ eine Methyl-, R-Aryl-, Heteroaryl-, $-COOR^4$-Gruppe ist, wobei $R^4$ Wasserstoff, $(C_1 - C_6)$-Alkyl, $R-C_6H_4-$ ist; wonach ein Methoxyphenol der Formel

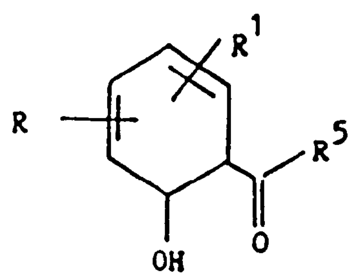

(II)

hinzugegeben wird,
wobei R und $R^1$ wie in Anspruch 1 definiert sind, man dann die Temperatur der Reaktionsmischung auf Raumtemperatur ansteigen läßt, wonach man die Verbindung

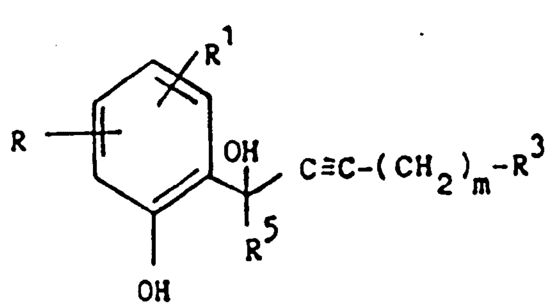

(IV)

die so erhalten wurde und bei der die Substituenten wie oben definiert sind, in Gegenwart von Methylenchlorid mit einer Mischung von Triethylsilan in etherischen Bortrifluorid bei einer Temperatur von -25° bis -15°C umsetzt, und man dann die Reaktionsmischung Raumtemperatur erreichen läßt.

3. Ein Verfahren zur Herstellung einer pharmazeutischen Zusammensetzung zur topischen Behandlung von Entzündungszuständen, dadurch gekennzeichnet, daß eine therapeutisch wirksame Menge einer Verbindung der Formel (I) nach Anspruch 1 mit einem geeigneten pharmazeutisch annehmbaren Verdünnungsmittel, Träger und/oder Arzneimittelträger vermischt wird.

**Patentansprüche für folgenden Vertragsstaat : GR**

1. Eine Verbindung der Formel

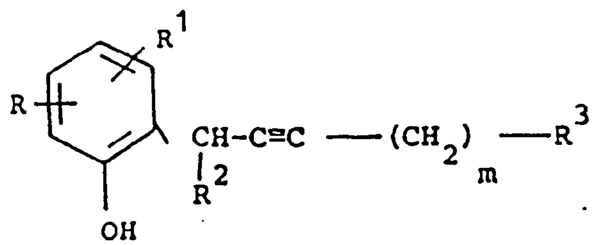

(I)

wobei
R und $R^1$ unabhängig voneinander ausgewählt werden aus Wasserstoff, Hydroxyl, Halogen, einer ($C_1$ - $C_6$)-Alkyl-, ($C_1$ - $C_4$)-Alkoxy-, Benzyloxy-, -$COOR^4$-Gruppe;
$R^2$ ist : Wasserstoff, Hydroxyl, ($C_1$ - $C_6$)-Alkyl, R-$C_6H_4$-;
$R^3$ ist eine Methyl-, R-Aryl-, Heteroaryl-, besonders Furyl- und Thienyl-, -$COOR^4$-Gruppe;
$R^4$ ist: Wasserstoff, ($C_1$ - $C_6$)-Alkyl, R-$C_6H_4$-;
m ist eine ganze Zahl von 0 bis 5;
und ein pharmazeutisch annehmbares Salz davon.

2. Eine Verbindung der Formel (I) nach Anspruch 1, dadurch gekennzeichnet, daß die Substituenten die folgenden Bedeutungen haben:
R und $R^1$: Wasserstoff, ($C_1$ - $C_4$)-Alkoxy, Benzyloxy;
$R^2$: Wasserstoff, Methyl;
$R^3$: Methyl, Phenyl;
m ist eine ganze Zahl von 0 bis 5; mit der Maßgabe, daß, wenn $R^3$ Phenyl ist, m dann 0 ist;
und ein pharmakologisch annehmbares Salz davon.

3. Ein Verfahren zur Herstellung einer Verbindung der Formel (I) nach Anspruch 1 oder 2, dadurch gekennzeichnet, daß eine Carbonylverbindung der Formel

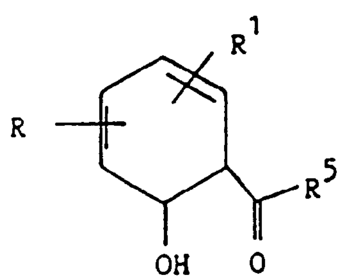

(II)

wobei R und $R^1$ wie in den vorhergehenden Ansprüchen definiert sind und $R^5$ Wasserstoff, ($C_1$ - $C_6$)-Alkyl oder R-$C_6H_4$- ist, bei einer Temperatur von -78°C bis zum Kochpunkt des(r) verwendeten Lösungsmittel(s) umgesetzt wird mit einem Alkalimetallsalz der Formel:

$$M^+ \, ^-C \equiv C - (CH_2)_m - R^3 \qquad (III)$$

wobei $R^3$ eine Methyl-, R-Aryl-, Heteroaryl-, -$COOR^4$-Gruppe ist, wobei $R^4$ Wasserstoff, ($C_1$ - $C_6$)-Alkyl,

$R-C_6H_4$- ist; gefolgt davon, daß man die Verbindung der Formel

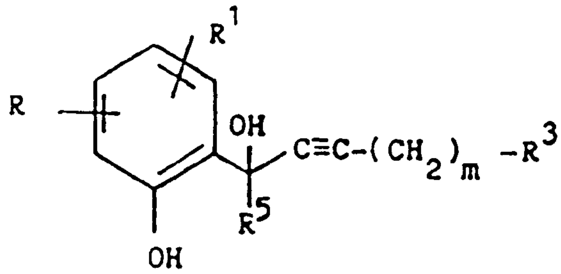

(IV)

die so erhalten wurde, wobei die Substituenten wie oben definiert sind, in Gegenwart eines organischen Lösungsmittels mit einer Mischung von Triethylsilan in etherischem Bortrifluorid bei einer Temperatur von -25° bis -15°C umsetzt, und man dann die Reaktionsmischung die Raumtemperatur erreichen läßt.

4. Ein Verfahren zur Herstellung einer Verbindung der Formel (I) nach Anspruch 1 oder 2, dadurch gekennzeichnet, daß das entsprechende Alkin in Tetrahydrofuran bei einer Temperatur von -78°C bis -75°C mit einem Reagenz umgesetzt wird, daß es umwandeln kann in das Acetylid

$$M^{+}\ {}^{-}C \equiv C - (CH_2)_m - R^3 \qquad \text{(III)}$$

wobei $R^3$ eine Methyl-, R-Aryl-, Heteroaryl-, -$COOR^4$-Gruppe ist, wobei $R^4$ Wasserstoff, ($C_1$ - $C_6$)-Alkyl, $R-C_6H_4$- ist;
wonach ein Methoxyphenol der Formel

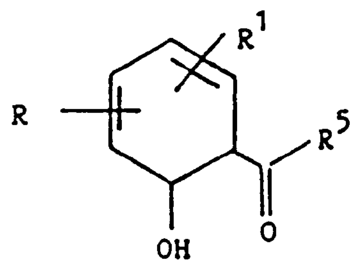

(II)

hinzugegeben wird,
wobei R und $R^1$ wie in den vorhergehenden Ansprüchen definiert sind und $R^5$ Wasserstoff, ($C_1$ - $C_6$)-Alkyl oder $R-C_6H_4$- ist, man dann die Temperatur der Reaktionsmischung auf Raumtemperatur ansteigen läßt, wonach man die Verbindung

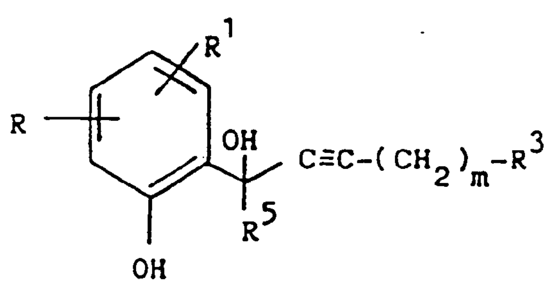

(IV)

die so erhalten wurde und bei der die Substituenten wie oben definiert sind, in Gegenwart von Methylenchlorid mit einer Mischung von Triethylsilan in etherischen Bortrifluorid bei einer Temperatur von -25° bis -15°C umsetzt, und man dann die Reaktionsmischung Raumtemperatur erreichen läßt.

5. Ein Verfahren zur Herstellung einer pharmazeutischen Zusammensetzung zur topischen Behandlung von Entzündungszuständen, dadurch gekennzeichnet, daß eine therapeutisch wirksame Menge einer Verbindung der Formel (I) nach Anspruch 1 mit einem geeigneten pharmazeutisch annehmbaren Verdünnungsmittel, Träger und/oder Arzneimittelträger vermischt wird.

## Revendications

**Revendications pour les Etats contractants suivants : AT, BE, CH, DE, DK, FR, GB, IT, LI, LU, NL, SE**

1. Composé de formule

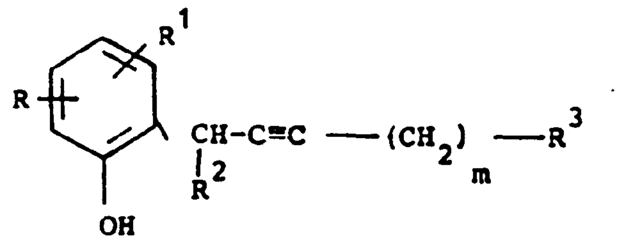

(I)

dans laquelle
R et R1 sont choisis indépendamment parmi : un atome d'hydrogène, un groupe hydroxyle, un atome d'halogène, ou un groupe alkyle en $C_1$-$C_6$, alcoxy en $C_1$-$C_4$, benzyloxy ou -$COOR^4$;
$R^2$ est un atome d'hydrogène, un groupe hydroxyle, ou un groupe alkyle en $C_1$-$C_6$ ou R-$C_6H_4$-;
$R^3$ est un groupe méthyle, R-aryle, hétéroaryle, en particulier furyle et thiényle, ou un groupe -$COOR^4$;
$R^4$ est un atome d'hydrogène, un groupe alkyle en $C_1$-$C_6$ ou R-$C_6H_4$-;
m est un nombre entier de 0 à 5;
et un sel pharmaceutiquement acceptable de ce composé.

2. Composé de formule (I) selon la revendication 1, caractérisé en ce que les substituants ont les significations suivantes :
R et $R^1$ : hydrogène, alcoxy en $C_1$-$C_4$, benzyloxy;
$R^2$ : hydrogène, méthyle;
$R^3$ : méthyle, phényle;
m est un nombre entier de 0 à 5; à condition que, lorsque $R^3$ est un groupe phényle, m soit nul, et un sel pharmaceutiquement acceptable de ce composé.

3. Procédé de préparation d'un composé de formule (I) selon la revendication 1 ou 2, caractérisé en ce que l'on fait réagir un composé carbonyle de formule

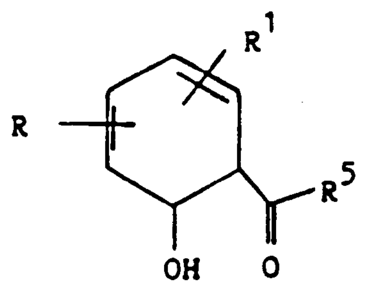

(II)

dans laquelle R et $R^1$ sont tels que définis dans les revendications précédentes et $R^5$ est un atome d'hydrogène, un groupe alkyle en $C_1$-$C_6$ ou R-$C_6H_4$-, à une température allant de -78°C au point d'ébullition du ou des solvants employés, avec un sel de métal alcalin de formule

$$M^+ \, {}^-C \equiv C - (CH_2)_m - R^3 \qquad (III)$$

dans laquelle $R^3$ est un groupe méthyle, R-aryle, hétéroaryle, ou un groupe -$COOR^4$; $R^4$ étant un atome d'hydrogène, un groupe alkyle en $C_1$-$C_6$ ou R-$C_6H_4$-;
puis on fait réagir le composé de formule

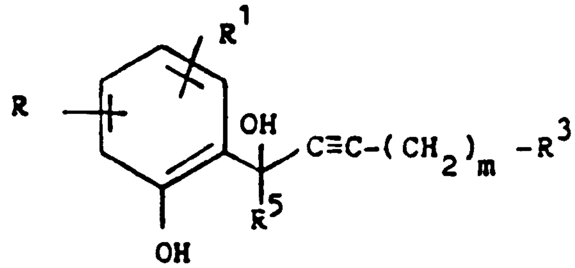

(IV)

ainsi obtenu, dans lequel les substituants sont tels que définis ci-dessus, en présence d'un solvant organique, avec un mélange de triéthylsilane et de trifluorure de bore éthéré, à une température de -25 à -15°C, puis on laisse le mélange réactionnel atteindre la température ambiante.

4. Procédé de préparation d'un composé de formule (I) selon la revendication 1 ou 2, caractérisé en ce que l'on fait réagir l'alcyne correspondant dans du tétrahydrofurane, à une température de -78°C-à -75°C, avec un réactif pouvant le transformer en acétylure

$$M^{+}\,^{-}C \equiv C - (CH_2)_m - R^3 \qquad (III)$$

dans lequel $R^3$ est un groupe méthyle, R-aryle, hétéroaryle, ou un groupe $-COOR^4$; $R^4$ étant un atome d'hydrogène, un groupe alkyle en $C_1$-$C_6$ ou R-$C_6H_4$-;
après quoi on ajoute un méthoxyphénol de formule

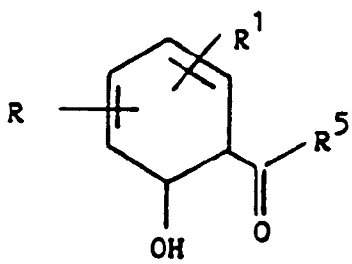

(II)

dans laquelle R et $R^1$ sont tels que définis dans les revendications précédentes et $R^5$ est un atome d'hydrogène, un groupe alkyle en $C_1$-$C_6$ ou R-$C_6H_4$-,
puis on laisse le mélange réactionnel atteindre la température ambiante, après quoi on traite le composé

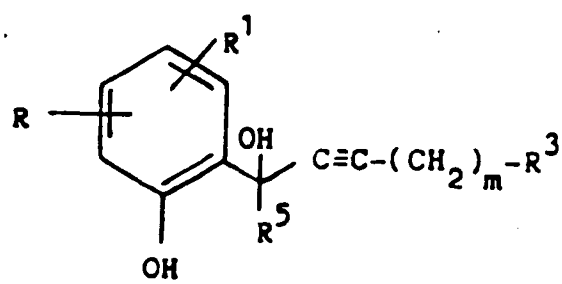

(IV)

ainsi obtenu, dans lequel les substituants sont tels que définis ci-dessus, en présence de chlorure de méthylène, avec un mélange de triéthylsilane et de trifluorure de bore éthéré, à une température de -25 à -15°C, puis on laisse le mélange réactionnel atteindre la température ambiante.

5. Composition pharmaceutique pour le traitement local d'états inflammatoires, caractérisée en ce qu'elle contient, comme agent à activité thérapeutique, une quantité pharmacologiquement efficace d'au moins un composé de formule (I) selon la revendication 1, ou sel pharmacologiquement acceptable de ce composé.

**Revendications pour l'Etat contractant suivant : ES**

1. Procédé de préparation d'un composé de formule

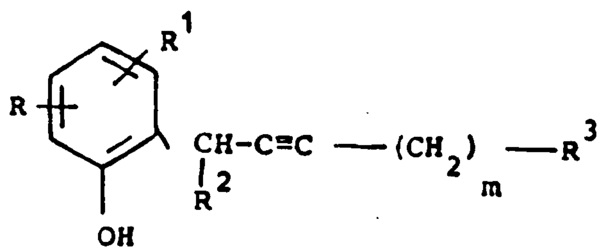

(I)

dans laquelle
R et $R^1$ sont choisis indépendamment parmi : un atome d'hydrogène, un groupe hydroxyle, un atome d'halogène, ou un groupe alkyle en $C_1$-$C_6$, alcoxy en $C_1$-$C_4$, benzyloxy ou -$COOR^4$;
$R^2$ est un atome d'hydrogène, un groupe hydroxyle, ou un groupe alkyle en $C_1$-$C_6$ ou R-$C_6H_4$-;
$R^3$ est un groupe méthyle, R-aryle, hétéroaryle, en particulier furyle et thiényle, ou un groupe -$COOR^4$;
$R^4$ est un atome d'hydrogène, un groupe alkyle en $C_1$-$C_6$ ou R-$C_6H_4$-;
m est un nombre entier de 0 à 5;
ou d'un sel pharmaceutiquement acceptable de ce composé, caractérisé en ce que l'on fait réagir un composé carbonyle de formule

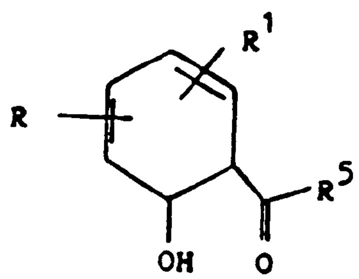

(II)

dans laquelle R et $R^1$ sont tels que définis ci-dessus et $R^5$ est un atome d'hydrogène, un groupe alkyle en $C_1$-$C_6$ ou R-$C_6H_4$-, à une température allant de -78°C au point d'ébullition du ou des solvants employés, avec un sel de métal alcalin de formule

$$M^+ \, {}^-C \equiv C - (CH_2)_m - R^3 \qquad (III)$$

dans laquelle $R^3$ est un groupe méthyle, R-aryle, hétéroaryle, ou un groupe -$COOR^4$; $R^4$ étant un atome d'hydrogène, un groupe alkyle en $C_1$-$C_6$ ou R-$C_6H_4$-;
puis on fait réagir le composé de formule

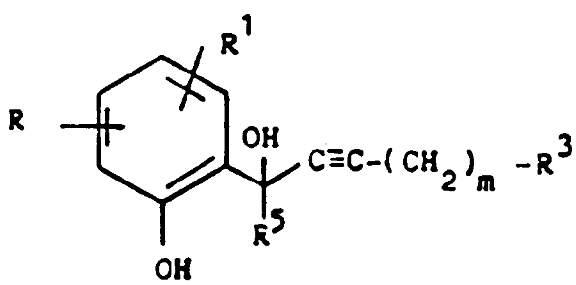

(IV)

ainsi obtenu, dans lequel les substituants sont tels que définis ci-dessus, en présence d'un solvant organique, avec un mélange de triéthylsilane et de trifluorure de bore éthéré, à une température de -25 à -15°C, puis on laisse le mélange réactionnel atteindre la température ambiante.

2. Procédé de préparation d'un composé de formule (I) selon la revendication 1, dans lequel
R et $R^1$ sont choisis indépendamment parmi un atome d'hydrogène, un groupe hydroxyle, un atome d'halogène, ou un groupe alkyle en $C_1$-$C_6$, alcoxy en $C_1$-$C_4$, benzyloxy ou -$COOR^4$;
$R^2$ est un atome d'hydrogène, un groupe hydroxyle, ou un groupe alkyle en $C_1$-$C_6$ ou R-$C_6H_4$-;
$R^3$ est un groupe méthyle, R-aryle, hétéroaryle, en particulier furyle et thiényle, ou un groupe -$COOR^4$;

$R^4$ est un atome d'hydrogène, un groupe alkyle en $C_1$-$C_6$ ou R-$C_6H_4$-;
m est un nombre entier de 0 à 5;
et d'un sel pharmaceutiquement acceptable de ce composé, caractérisé en ce que l'on fait réagir l'alcyne correspondant dans du tétrahydrofurane, à une température de -78°C à -75°C, avec un réactif pouvant le transformer en acétylure

$$M^+ \, {}^-C \equiv C - (CH_2)_m - R^3 \qquad (III)$$

dans lequel $R^3$ est un groupe méthyle, R-aryle, hétéroaryle, ou un groupe -$COOR^4$; $R^4$ étant un atome d'hydrogène, un groupe alkyle en $C_1$-$C_6$ ou R-$C_6H_4$-;
après quoi on ajoute un méthoxyphénol de formule

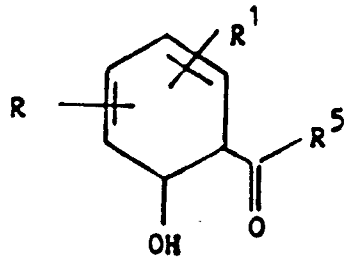

(II)

dans laquelle R et $R^1$ sont tels que définis dans la revendication 1, puis on laisse le mélange réactionnel atteindre la température ambiante, après quoi on traite le composé

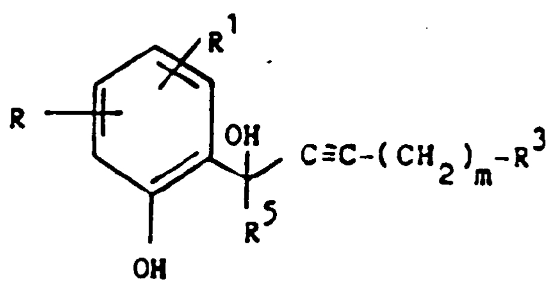

(IV)

ainsi obtenu, dans lequel les substituants sont tels que définis ci-dessus, en présence de chlorure de méthylène, avec un mélange de triéthylsilane et de trifluorure de bore éthéré, à une température de -25 à -15°C, puis on laisse le mélange réactionnel atteindre la température ambiante.

3. Procédé de préparation d'une composition pharmaceutique pour le traitement local d'états inflammatoires, caractérisé en ce que l'on mélange une quantité à activité thérapeutique d'un composé selon la revendication 1 avec un diluant, un support et/ou un excipient pharmaceutiquement acceptable et convenable.

**Revendications pour l'Etat contractant suivant : GR**

1. Composé de formule

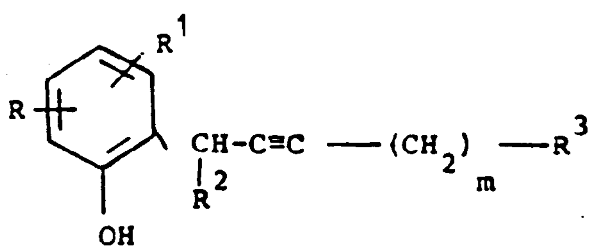

(I)

dans laquelle
R et $R^1$ sont choisis indépendamment parmi : un atome d'hydrogène, un groupe hydroxyle, un atome d'halogène, ou un groupe alkyle en $C_1$-$C_6$, alcoxy en $C_1$-$C_4$, benzyloxy ou -$COOR^4$;
$R^2$ est un atome d'hydrogène, un groupe hydroxyle, ou un groupe alkyle en $C_1$-$C_6$ ou R-$C_6H_4$-;
$R^3$ est un groupe méthyle, R-aryle, hétéroaryle, en particulier furyle et thiényle, ou un groupe -$COOR^4$;
$R^4$ est un atome d'hydrogène, un groupe alkyle en $C_1$-$C_6$ ou R-$C_6H_4$-;
m est un nombre entier de 0 à 5;
et un sel pharmaceutiquement acceptable de ce composé.

2. Composé de formule (I) selon la revendication 1, caractérisé en ce que les substituants ont les significations suivantes :
R et $R^1$ : hydrogène, alcoxy en $C_1$-$C_4$, benzyloxy;
$R^2$ : hydrogène, méthyle;
$R^3$ : méthyle, phényle;
m est un nombre entier de 0 à 5; à condition que, lorsque $R^3$ est un groupe phényle, m soit nul, et un sel pharmaceutiquement acceptable de ce composé.

3. Procédé de préparation d'un composé de formule (I) selon la revendication 1 ou 2, caractérisé en ce que l'on fait réagir un composé carbonyle de formule

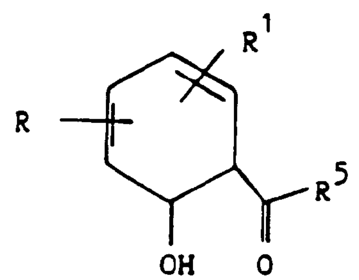

(II)

dans laquelle R et $R^1$ sont tels que définis dans les revendications précédentes et $R^5$ est un atome d'hydrogène, un groupe alkyle en $C_1$-$C_6$ ou R-$C_6H_4$-, à une température allant de -78°C au point d'ébullition du ou des solvants employés, avec un sel de métal alcalin de formule

$$M^+ \, ^-C \equiv C - (CH_2)_m - R^3 \qquad (III)$$

dans laquelle $R^3$ est un groupe méthyle, R-aryle, hétéroaryle, ou un groupe -$COOR^4$; $R^4$ étant un atome d'hydrogène, un groupe alkyle en $C_1$-$C_6$ ou R-$C_6H_4$-;
puis on fait réagir le composé de formule

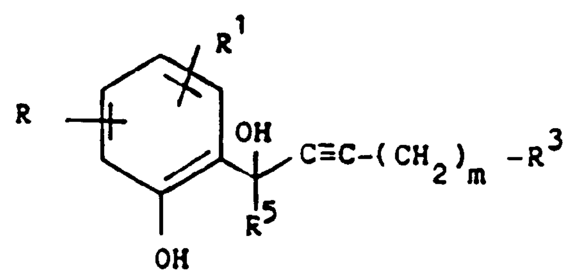

(IV)

ainsi obtenu, dans lequel les substituants sont tels que définis ci-dessus, en présence d'un solvant organique, avec un mélange de triéthylsilane et de trifluorure de bore éthéré, à une température de -25 à -15°C, puis on laisse le mélange réactionnel atteindre la température ambiante.

4. Procédé de préparation d'un composé de formule (I) selon la revendication 1 ou 2, caractérisé en ce que l'on fait réagir l'alcyne correspondant dans du tétrahydrofurane, à une température de -78°C à -75°C, avec un réactif pouvant le transformer en acétylure

$$M^+ \, ^-C \equiv C - (CH_2)_m - R^3 \qquad (III)$$

dans lequel $R^3$ est un groupe méthyle, R-aryle, hétéroaryle, ou un groupe -$COOR^4$; $R^4$ étant un atome d'hydrogène, un groupe alkyle en $C_1$-$C_6$ ou R-$C_6H_4$-;
après quoi on ajoute un méthoxyphénol de formule

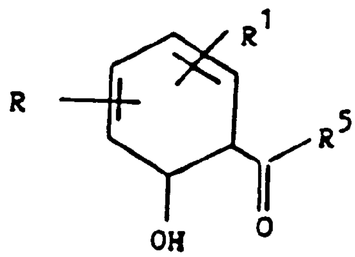

(II)

dans laquelle R et $R^1$ sont tels que définis dans les revendications précédentes et $R^5$ est un atome d'hydrogène, un groupe alkyle en $C_1$-$C_6$ ou R-$C_6H_4$-,
puis on laisse le mélange réactionnel atteindre la température ambiante, après quoi on traite le composé

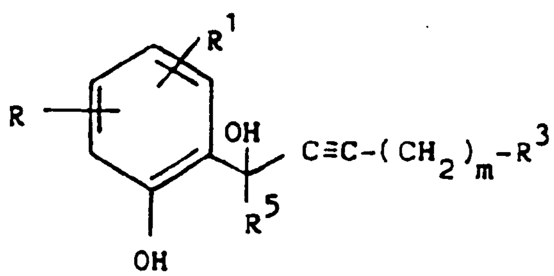

(IV)

ainsi obtenu, dans lequel les substituants sont tels que définis ci-dessus, en présence de chlorure de méthylène, avec un mélange de triéthylsilane et de trifluorure de bore éthéré, à une température de -25 à -15°C, puis on laisse le mélange réactionnel atteindre la température ambiante.

**5.** Procédé de préparation d'une composition pharmaceutique pour le traitement local d'états inflammatoires, caractérisé en ce que l'on mélange une quantité à activité thérapeutique d'un composé selon la revendication 1 avec un diluant, un support et/ou un excipient pharmaceutiquement acceptable et convenable.